# EUROPEAN PATENT SPECIFICATION

(11) **EP 2 642 965 B1**
(45) Date of publication and mention of the grant of the patent: **04.11.2015**
(21) Application number: 11784687.3
(22) Date of filing: 21.11.2011
(51) Int. Cl.: A61J 1/20, A61M 5/31

(54) **ADAPTER**
ADAPTER
ADAPTATEUR

(30) Priority: 22.11.2010 EP 10192031
(43) Date of publication of application: 02.10.2013
(73) Proprietor: Novartis AG, 4056 Basel (CH)
(72) Inventor: GAILLOT, Maxime, CH-4002 Basel (CH); FARRELL, Kathryn, Cambridge Cambridgeshire CB4 0DW (GB); GOW, Andrew, Cambridge Cambridgeshire CB4 0DW (GB); PERKINS, George, Cambridge Cambridgeshire CB4 0DW (GB)
(74) Representative: Stuttle, James Henry Phillip
(86) International application number: PCT/EP2011/070531
(87) International publication number: WO 2012/069401

(56) References cited:
- GB-A- 2 446 778

## Description

The present invention relates to an adapter for the transfer of a fluid from a storage container to a destination reservoir. Specifically the invention relates to an adapter for coupling a syringe to a vial to allow fluid transfer between the vial and syringe.

It is known that some medicaments are not sufficiently stable in a liquid formulation to be stored in that liquid form and subsequently delivered to a patient. In such cases there is an option to supply the medicament in a solid form, such as dried or lyophilised, together with a liquid. Prior to use the liquid can be added to the medicament to reconstitute the medicament into a useable, for example injectable, or at least more convenient liquid form.

Some medicament kits use a vial to store a dried medicament and a syringe to store a reconstitution liquid. In order to reconstitute the medicament a needle attached to the syringe is used to pierce a stopper sealing the vial and the liquid transferred into the vial. The vial can then be agitated to achieve a substantially homogeneous solution and a dose withdrawn from the vial for subsequent injection. The subsequent injection could use the needle previously used for the reconstitution operation, but this needle is likely to have been dulled during the stopper piercing and so might need replacing in order to provide satisfactory skin piercing performance. The manual nature of the reconstitution can also lead to user errors and thus unexpected performance.

It is known, for example from GB2446778 or US 3,940,003, to provide an adapter between a syringe and vial.

The present invention, as defined by the appended claims, provides an adapter, the adapter comprising a base having a container attachment portion on a first side thereof and a needle hub attachment portion on a second side thereof, the first side being opposite the second side, the adapter including a puncturing member extending along a first axis for entering a container inserted into the container attachment portion, the adapter including a channel extending substantially along the first axis from the needle hub attachment portion, through the base and along the puncturing member to an outlet at, or adjacent, a tip of the puncturing member, the adapter configured such that a needle comprising a shaft extending from a hub can be inserted into the adapter such that the hub engages the hub attachment portion and the shaft extends through the base and within the channel, the adapter characterised in that the outlet is an axial outlet from the puncturing member, the axial outlet extending substantially along the first axis and the needle shaft extends within the channel towards the axial outlet, wherein the container attachment portion is adapted to snap fit to a vial and the puncturing member is sharpened such that it will penetrate an elastomeric vial stopper sealing the vial as the vial is inserted into the container attachment portion and in which the needle hub attachment portion is adapted to releasably couple to a needle hub inserted therein, the needle hub attachment portion including a seal for sealing to the needle hub inserted therein.

The adapter is intended to be arranged between a first container, for example a vial, and a needle hub and is configured such that a needle comprising a shaft extending from a hub can be inserted into the adapter such that the hub engages the hub attachment portion and the shaft extends through the base and within the puncturing member towards the axial outlet. Using such an adapter it is possible to force liquid through the needle shaft, for example from a syringe attached to the needle hub, so that it forms a spray. Since the axial outlet from the puncturing member extends substantially along the first axis the spray emitted from the needle shaft can passes directly through the axial outlet of the puncturing member and into the container. Such a spray entering the container is likely to impact one or more walls of the container and be deflected within the container. The spray impacting on one or more walls of the container leads to significant wall wetting and spray redirection and therefore facilitates reconstitution. The use of such a spray during reconstitution may reduce any orientation sensitivity in the initial stages of a reconstitution process.

The container may be any suitable container for a solid medicament to be reconstituted, for example a lyophilised or dried powder, such as a bottle, flask or flexible pouch. In one embodiment the container is a vial, for example a glass vial. The vial may be sealed by an elastomeric stopper which can be punctured by the puncturing member to permit a tip of the puncturing member to enter the vial.

The puncturing member extends along a first axis and is adapted to enter a container coupled to the adapter, this may be by piercing or puncturing through a portion of the container, for example a seal or wall. The puncturing member may comprise an elongate body which has a substantially constant cross section shape, or which may taper so that the body narrows away from the base. The puncturing member may also include a piercing tip which may be sharpened to facilitate piercing. The piercing tip may be pointed or include one or more bevels or facets to provide a sharp tip, for example similar to that of a pin, needle or hypodermic needle. In one embodiment the cross section shape of the body of the puncturing member is substantially circular and the piercing tip is a plain bevel.

A channel extends substantially along the first axis from the needle hub attachment portion, through the base and along the puncturing member to an axial outlet at, or adjacent, a tip of the puncturing member. The channel may have a circular cross section. The diameter of the channel is adapted to be larger than the outer diameter of a needle shaft intended for use with the adapter so that the shaft can extend within the channel. To avoid significant liquid dead volume in use the channel diameter may be less than 50% larger than needle shaft outer diameter and may be less than 25% larger or less than 10% larger. The channel diameter should be small to reduce dead volume, but large enough so that insertion of the needle into, or removal of the needle from, the channel does not result in needle damage. To reduce the dead volume the end of the needle shaft should extend substantially the full length of the channel. The needle shaft may extend to within 1cm of the axial outlet, to within 5mm of the axial outlet and may extend to within 2mm of the axial outlet. This reduces dead volume within the device and also allows the spray from the needle to exit substantially intact from the axial outletto obtain a suitable spray.

The adapter includes a needle hub attachment portion which is adapted to releasably couple to a needle hub. The adapter therefore provides a means for enabling fluid transfer via a needle between a container of a medicament product and a second container attached to the needle hub. In one embodiment the second container is a syringe.

A needle may be permanently attached to the syringe such that the needle hub is integrally formed with a body of the syringe. In another embodiment the needle may be formed separately and the needle hub may be configured to be coupled to a syringe by any suitable mechanism, for example by a well known luer lock mechanism. The needle comprises a needle hub and a needle shaft. The needle shaft includes a bore extending therethrough and is coupled to the needle hub. The needle hub is connected to, or allows connection to a further container such as a syringe. The needle may include a sharpened piercing tip, for example one of the many standard hypodermic tips suitable for piercing skin.

By using a needle to provide the spray into the container, rather than, for example relying on the axial outlet of the puncturing member to generate the spray the requirement to finely control the diameter of axial outlet in the puncturing member is removed so the tolerances during the adapter need not be controlled so strictly which facilitates adapter manufacture. A needle, for example a hypodermic needle, is a well known piece of apparatus and can be obtained from many sources. Such needles can be specified to have particular needle shaft outer diameters, needle bore sizes, needle shaft lengths and needle hub configurations. The tolerances to which such needles are manufactured are suitable for reliable spray generation through the needle shaft and the inclusion of a needle in this way may have particular patient use benefits which will be discussed below, particularly if the needle can be used not only for spray generation and fluid transport during reconstitution, but also for subsequent injection. It is also noted that the typically small bores of such needles may introduce additional turbulence into the solution during the reconstitution process and thereby enhance mixing within the solution.

By providing an axial outlet on the first axis, an outlet that is orientated so that it is substantially parallel with the first axis, in the puncturing member the spray from the needle can be directly utilised to facilitate reconstitution. This is contrary to other reconstitution devices which seek to diffuse liquid entering the container in an apparent effort to reduce spray or a risk of poor liquid distribution and also to reduce any risk of foaming, or bubble formation within the reconstituted liquid. This is the case in, for example GB 2446778, in which the spray from a needle in the adapter cannot pass directly from the needle into the vial as a solid puncture portion is located on the central axis of the needle so spray from the needle contacts this puncture portion before it can exit through one or more ports located radially offset from the central axis. The spray from the needle is therefore significantly slowed by the time it enters the vial. It has been found that using a direct spray from a needle to enter the vial in fact enhances reconstitution and if there is any problem with foaming, or bubble formation the spray velocity can be adjusted and the problem avoided. This adapter is therefore particularly suitable for automatic reconstitution devices in which the spray velocity can be closely controlled to be rapid, but not fast enough to risk foaming. The exact speed will depend upon many things including the liquid and solid involved, the needle bore and the container shape, but can be readily determined during tests.

The needle extends within the channel in the puncturing member and does not protrude from the end thereof. This means that if, for example, the container is a vial with an elastomeric stopper the puncturing member punctures the stopper and not the needle. This means that the needle tip is not blunted to gain access to the interior of the container and any silicone coated onto the needle shaft to reduce pain during a subsequent injection is not removed. In this way the puncturing member acts as a needle shield and allow the needle to be used not only for reconstitution, but also for a subsequent injection.

The puncturing member may be adapted such that, when inserted through an elastomeric stopper, the tip of the puncturing member does not protrude too far into the container so that the axial outlet is near the internal wall of the stopper. Having the axial outlet near the stopper means that, when the assembly is inverted with that the vial is above the needle the axial outlet can be used to withdraw the largest amount of solution. When installed, the axial outlet may be less than 10mm from the stopper, may be less than 5mm from the stopper or may be less than 3mm from the stopper.

The container attachment portion is adapted to permit releasable coupling to a container, such as a vial, the container attachment portion comprises snap fit features intended to couple the adapter to the container, for example a vial, and substantially hinder subsequent removal. The snap fit features comprise resiliently biased legs which include shoulders or ridges directed towards the base such that as the container is coupled to the adapter the legs are forced away from the puncturing member until the shoulder passes a corresponding shoulder or ridge at which point the legs are biased back towards their original position such that the shoulders and or ridges cooperate to substantially hinder removal of the container from the adapter.

The needle hub attachment portion includes a seal to seal to the needle hub so as to create a substantially fluid tight engagement between the adapter and the needle hub. The seal may be arranged at or adjacent the connection between the needle shaft and the hub as this reduces dead volume within the apparatus in use. The seal may be an 'O' ring seal arrange in the adapter or any other suitable seal.

The adapter may further include a resiliently biased flag. The adapter can then be arranged such that the flag is biased to a first position when no container is coupled to the adapter and which is forced to a second position when a container is coupled to the container attachment portion. This flag may comprise a projecting member the position of which can be detected by a detector that forms part of a device to which the adapter, needle and container assembly is to be coupled. This would allow the device to determine whether or not a vial is present in the adapter without having to detect or contact the container itself. Additionally, or alternatively, the projection member may substantially prevent coupling of the adapter to the device when in the first position and permit coupling when in the second position so that the adapter can only be coupled to the device when a container is present. In one embodiment the device is an automatic reconstitution apparatus. In another embodiment the device is an automatic reconstitution apparatus which can also perform an injection step. In yet another embodiment the device is a syringe with an adapter to prevent the syringe coupling to the needle in the assembly when there is no container present in the adapter.

The adapter may be manufactured as a single member or as a plurality of separate parts that are coupled together. The, or each, part may be moulded, etched, or otherwise formed. The parts need not all be formed in the same way. If more than one part is used to construct the adapter then parts may be connected together in any suitable manner, for example glued, welded, push fit, snap fit, held with connectors such as screws or rivets, or any other suitable manner. The adapter may further include ribs on an outer surface to facilitate gripping of said surface by a user.

The needle may include one or more engagement features on the hub which are adapted to engage with corresponding features of the hub attachment portion facilitating transfer of torque substantially about the first axis between the adapter and needle.

The invention also provides an adapter assembly comprising a needle and an adapter. The adapter is as described above and the needle comprises a hub and a shaft, the shaft extending from the hub. The needle is suitable for performing a subcutaneous injection when coupled to a syringe and the needle hub is releasably coupled to the needle hub attachment portion of the adapter.

A patient kit of a syringe and vial can be supplied to a patient with such an assembly to allow them to perform a manual reconstitution and subsequent injection if desired. Alternatively, the same kit or syringe, vial and adapter assembly can be supplied and fitted into a reusable reconstituting auto-injector apparatus allowing for an automated reconstitution and injection process using the standard kit.

The invention also provides a method of reconstituting a medicament comprising providing a container containing a medicament to be reconstituted, a syringe containing a liquid, a needle comprising a hub and a shaft, the hub being coupled to the syringe and an adapter as described above, the method comprising the steps of:
arranging the needle within the adapter such that the hub engages the hub attachment portion and the shaft extends through the base and within the channel towards the outlet;
coupling the container to the container attachment portion such that the puncturing member enters the container to create a reconstitution assembly; using the syringe to force fluid through the needle shaft to cause liquid to spray from the shaft, through the axial outlet in the puncturing member and into the container.

As noted above the spray emitted from the needle into the container can improve container wall wetting and therefore improve reconstitution reliability. The method may further include, after spraying the liquid into the container, the steps of arranging the reconstitution assembly with the container above the needle and using the syringe to draw the solution from the container into the syringe. The syringe may then be used to force the solution back into the container through the needle. By forcing the solution back and forth through the needle and between two different containers the mixing within the solution is enhanced and the reconstitution process can be more reliable.

The invention also extends to a kit of parts for reconstituting and delivering a medicament, the kit comprising a container containing a medicament to be reconstituted, a syringe containing a liquid, a needle comprising a hub and a shaft, the hub being coupled to the syringe and an adapter comprising a base having a container attachment portion on a first side thereof and a needle hub attachment portion on a second side thereof, the first side being opposite the second side, the adapter including a puncturing member extending along a first axis, the adapter including a channel extending substantially along the first axis from the needle hub attachment portion, through the base and along the puncturing member to an axial outlet at, or adjacent, a tip of the puncturing member.

The invention also provides an adapter, the adapter comprising a base having a container attachment portion on a first side thereof and a needle hub attachment portion on a second side thereof, the first side being opposite the second side, the adapter including a puncturing member extending along a first axis for entering a container inserted into the container attachment portion, the adapter further includes a resiliently biased flag, the adapter is arranged such that the flag is biased to a first position when no container is coupled to the adapter and which is forced to a second position when a container is coupled to the container attachment portion.

It should be understood that throughout this specification and in the claims that follow, unless the context requires otherwise, the word "comprise", or variations such as "comprises" or "comprising", implies the inclusion of the stated integer or step, or group of integers or steps.

The invention will now be further described, by way of example only, with reference to the following drawings in which:
Figure 1 shows a picture of an assembly comprising a needle and an adapter;
Figure 2 shows a cross section through the assembly of Figure 1;
Figure 3 shows a vial being inserted into the assembly of Figure 1 to create an assembly to be inserted into a device;
Figure 4 shows an assembly of a comprising a needle, an adapter and a vial;
Figures 5a to 5e show a reconstitution sequence;
Figures 6a to 6c show an initial preparation sequence; and
Figure 7 shows a syringe ready for use following reconstitution; and
Figure 8 shows a further embodiment of an adapter.

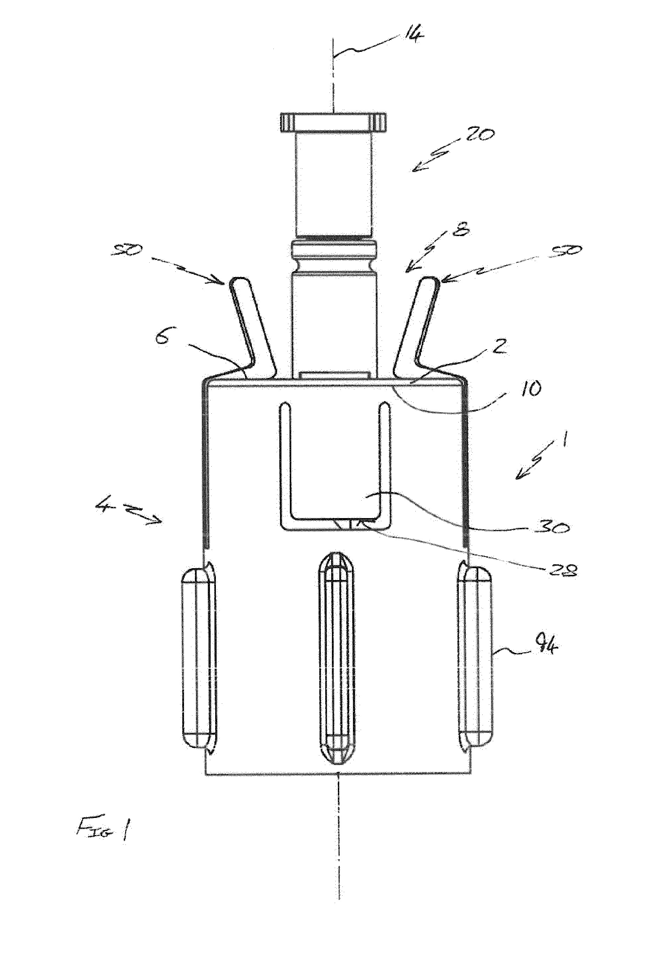

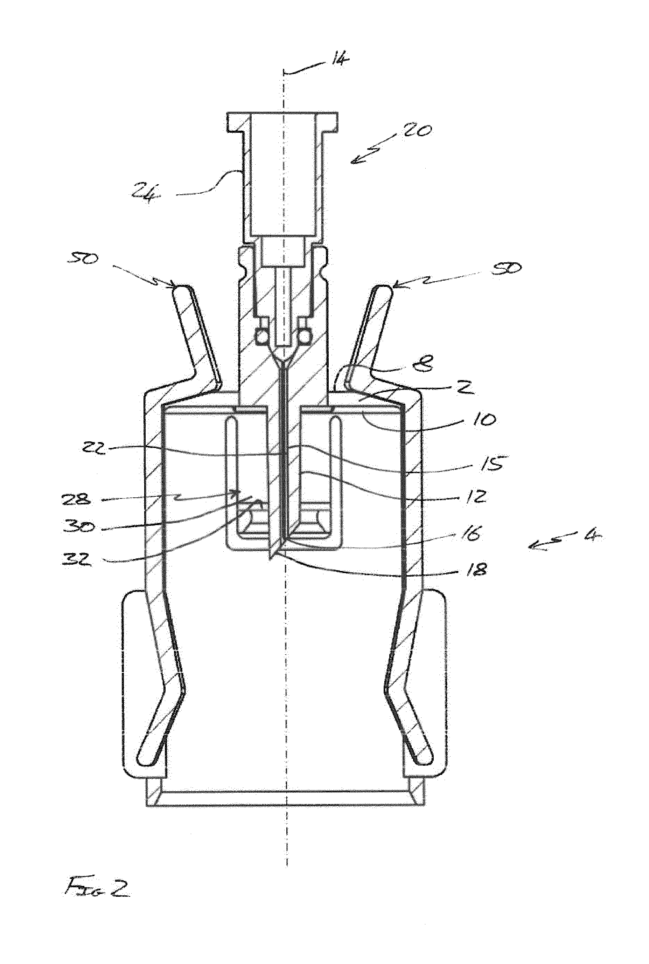

Figures 1 and 2 show an adapter 1 and a needle 20 coupled together. The adapter comprises a base 2 having a container attachment portion 4 on a first side 6 thereof and a needle hub attachment portion 8 on a second side 10. The first side 6 is opposite the second side 10. The adapter 1 includes a puncturing member 12 extending along a first axis 14 for entering a container 16 inserted into the container attachment portion 8. The adapter 1 includes a channel 15 extending substantially along the first axis 14 from the needle hub attachment portion 8, through the base 2 and along the puncturing member 12 to an axial outlet 16 at, or adjacent, a tip 18 of the puncturing member 12. In this case the tip 18 comprises a substantially plain bevel end cut and the channel 15 extends substantially through the centre of the puncturing member 12.

The needle 20 comprises a shaft 22 extending from a hub 24. The hub 24 is configured for attachment to a syringe (not shown in this Figure) using, for example a luer lock adapter.

The adapter 1 is configured such that, when the needle 20 is coupled to the adapter 1 the hub 24 engages the hub attachment portion 8 and the shaft 22 extends within the channel 15 through the base 2 and within the puncturing member 12.

The hub attachment portion 8 also includes a seal 26, in this case an 'O'-ring seal, which engages the needle hub 24 adjacent the connection between needle hub 24 and needle shaft 22 to provide a substantially fluid tight connection. In other embodiments an 'O' ring, or other seal forming region or member, may be arranged on the needle hub and seal onto a seal surface in the hub attachment portion.

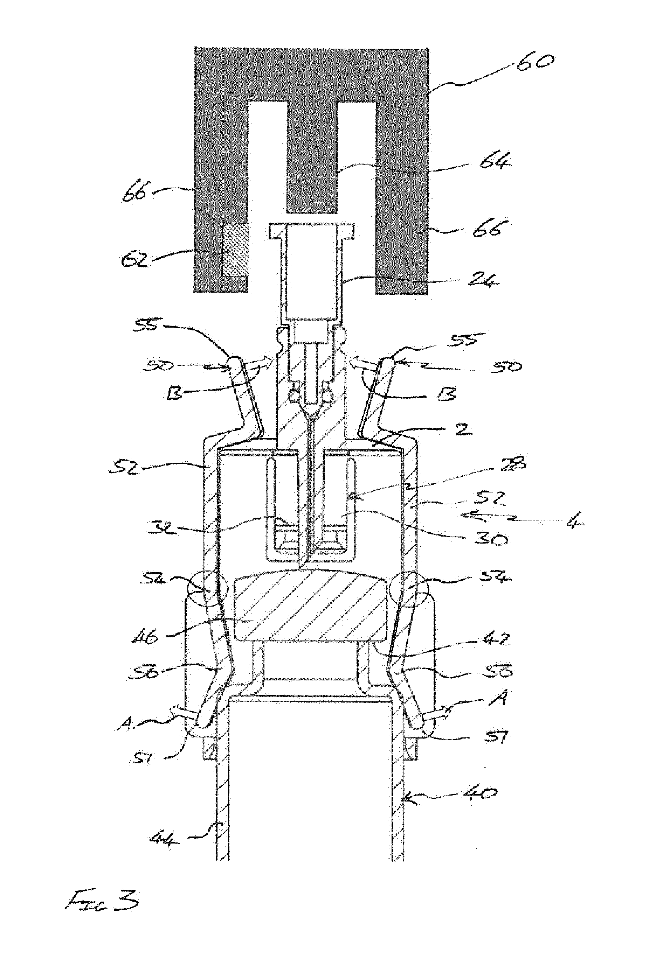

As best shown in Figure 3, the container attachment portion 4 comprises snap fit features 28 comprising resiliently deformable legs 30 which include inwardly protruding shoulders 32 directed towards the base 2. The snap fit features 28 are adapted such that as a container 40 is coupled to the adapter 1 the legs 30 are forced outward, away from the puncturing member 12, until the shoulder 32 passes a corresponding outwardly protruding shoulder 42 on the container 40. Once passed that point, the legs 30 are biased back towards their original position such that the shoulders 32, 42 cooperate to substantially hinder removal of the container 40 from the adapter 1. The container 40 comprises a glass vial 44 which is sealed at a top end by an elastomeric stopper 46.

Figure 3 shows a vial 44 sealed with an elastomeric stopper 46 being inserted into the container attachment portion 4. Figure 3 also shows how the vial 44 will interact with the adapter 1 and flags 50 provided thereon. The adapter 1 includes two flags 50 which, in this embodiment are levers 52 pivoted about a hinge point 54 and are resiliently biased to a first position as shown in this Figure. In this case the levers 52 are integrally formed, in this case moulded, with the adapter 1. Adjacent a first end 51 of the levers 52 there is a projection 56 directed into the container attachment portion 4. The second end 55 of the lever 52 extends beyond the base 2, and in the first position (as shown) is located a first distance away from the first axis 14.

The projections 56 and levers 54 are configured such that as the vial 44 is inserted into the adapter 1 the projections are forced outward by the vial 44, or possibly the stopper 42. As the projections 56 are forced outward (as indicated by the arrows A), the lever 52 pivots about the hinge 54 causing a second end 55 of the lever 52 opposite the first end 51 to move inwards (as indicated by the arrows B), towards the first axis 14 so that, when the vial 44 is fully inserted and held in place by the snap fit features 28 the flags 50 are in a second position in which the second ends are located a second distance from the first axis 14. The second distance is smaller than the first distance.

Since the flags 50 have moved in response to the presence of the vial 44 in the adapter 1 a device 60 into which the adapter assembly it to be inserted includes a sensor 62 to detect the presence of the flags 50 in the second position in order to confirm that a vial 44 is present. In other embodiments a sensor may be used to detect the presence of a portion of the adapter or of a portion of the vial or vial seal itself. In yet further embodiments, one or more sensors arranged at or adjacent an attachment area of a device to which the adapter is to be attached may be used to detect the presence of one or more of a flag on the adapter, a portion of the adapter, a portion of the vial or stopper.

In this embodiment the device 60 also includes blocking members 66 which prevent the device 60 from making a complete connection with the adapter 1 and needle 20 assembly by contact between the second end 55 of the levers 52 and the blocking members 66 which the flags 50 are in the first position, indicating that no container 40 is inserted into the adapter 1. When the flags 50 are in the second position, indicating that a container 40 is inserted into the adapter 1, the flags 50 are able to pass the blocking members 66 to enable a complete connection with the adapter 1 and needle 20 assembly.

The device 60, in this case a reconstituting auto-injector, includes a luer lock adapter 64 to engage with the corresponding luer lock adapter in the needle hub 24.

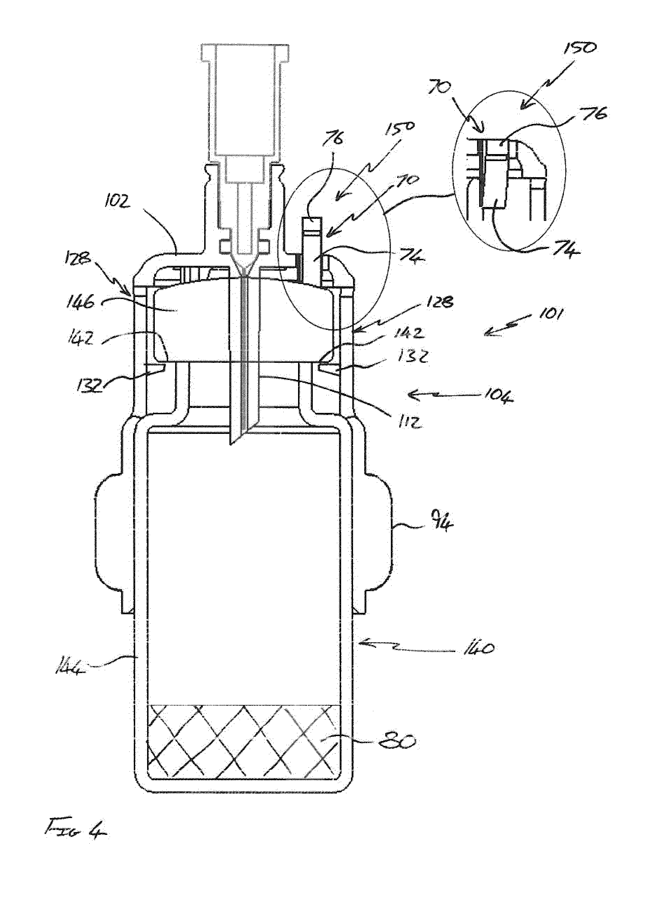

Figure 4 shows a different embodiment of an adapter 101 to that of the previous Figures. Similar reference numerals will be used for similar parts, but with the numbers incremented by 100.

In this Figure a container 140, in this case a vial 144 sealed by an elastomeric stopper, is shown inserted into the container attachment portion 104 of the adapter 101. The puncturing member 112 has pierced through the elastomeric stopper 146 such that the axial outlet lies close to the stopper 146 within the container 140. The container 140 includes a lyophilised medicament product 80.

The shoulders 132 of the snap fit features 128 are located adjacent the corresponding shoulders 142 on the vial 144.

The adapter 101 includes a flag 150 which in this embodiment comprises a resiliently biased lever 70 arranged in the base 102. The lever 70 is biased to a first position (shown in the inset figure) in which the lever 70 lies substantially in the plane of the base 102. The lever 70 is coupled to the base 102 at a first end (not shown) and includes at a second end a visual member 76 and a projection 74 that extends through the base 102 and into the container attachment portion 104. In the first position (not shown) the visual member 76 is substantially hidden within the base 102. When a container 140 is inserted into the adapter 101 a portion of the container 140 makes contact with the projection 74 and forces the lever 70 out of the plane of the base 102 and into a second position as shown in the main image. In the second position the visual member 76 is no longer hidden within the adapter 101 and can be detected by a device into which the adapter 101 is to be inserted.

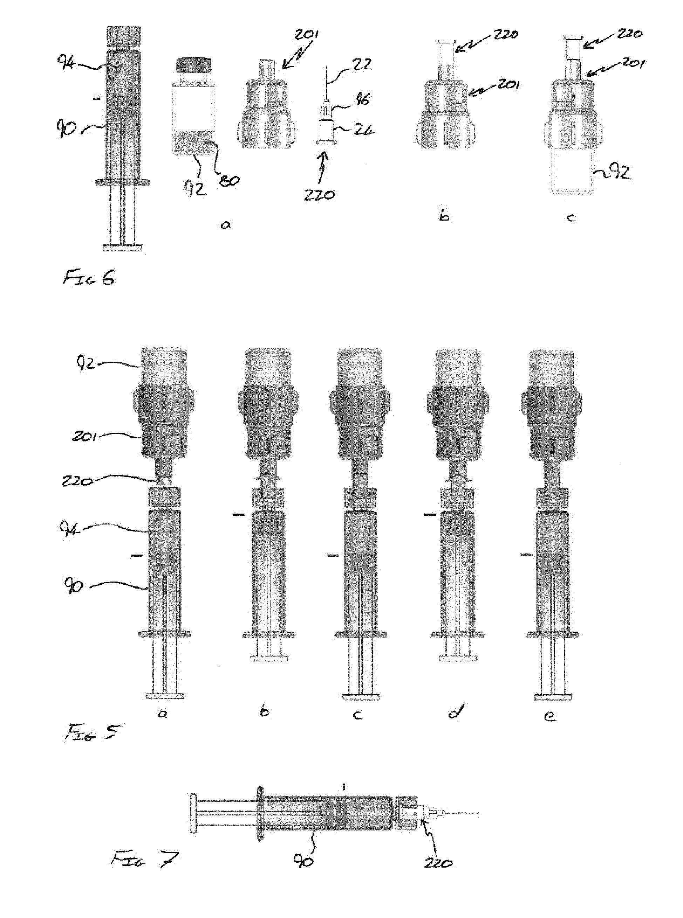

Figure 5 shows a sequence of steps in a reconstitution method using an adapter 201. Figure 5 shows a yet another embodiment of an adapter 201 to that of the previous Figures. Similar reference numerals will be used for similar parts, but with the numbers incremented by 100 from the embodiment shown in Figures 1 to 3. The adapter is essentially the same as that shown in Figure 2, but does not include flags.

Prior to Figure 5a a kit of parts is provided which comprises an adapter 201 and a needle 220. The needle 220 is inserted into the adapter 201 as described in connection with Figures 1 and 2 such that a shaft of the needle lies within a channel extending through a puncturing member of the adapter 201. The kit of parts further comprises a syringe 90 and a vial 92. The vial 92 contains a lyophilised medicament product 80. The syringe 90 contains a reconstitution liquid 94 and includes a luer lock adapter at a front end.

In preparation for reconstitution the vial 92 is inserted into the container attachment portion of the adapter 201. As the vial 92 is inserted the puncturing member pierces through an elastomeric stopper which seals the vial so that the axial outlet of the puncturing member is located within the container adjacent the stopper.

The syringe 90 is then coupled to the luer lock adapter of the hub of the needle 220 to form the assembly as shown in Figure 5a which is then arranged with the vial 92 above the syringe 90. The syringe 90 is then used to force substantially the entire original volume of liquid into the vial as shown in Figure 5b. As the liquid is forced from the syringe 90 into the vial 92 it passes through the needle 220 and is sprayed from the end of the needle shaft such that the spray passes through the axial outlet and impacts on a wall of the vial 92 causing wall wetting and spray redirection which assists with the initial dissolution of the lyophilised medicament 80 to form an initial solution within the vial 92.

Some of the initial solution is then drawn back through the needle into the syringe as shown in Figure 5c. The amount withdrawn from the vial is less than the amount initially forced into the vial as this reduces the likelihood that air will be sucked into the syringe and cause bubbling. The amount withdrawn back into the syringe may be less than 90% of the original volume of liquid, may be less than 80%, or less than 75% of the original volume. The turbulence caused during the withdrawing action further assists mixing and dissolution. The solution is then sprayed back into the vial 92 to further mix the solution and reduce the likelihood that any medicament remains in a solid form (Figure 5d). This withdraw and spray process may be repeated if desired, but it has been found that spraying the initial solution back into the vial a single time is sufficient to achieve reliable reconstitution.

The desired amount of solution is then withdrawn into the syringe 90 to provide the injection solution as shown in Figure 5e. Once the injection solution is within the syringe 90 the syringe 90 and needle 220 assembly is withdrawn from adapter 201 and the syringe 90 can be used for an injection.

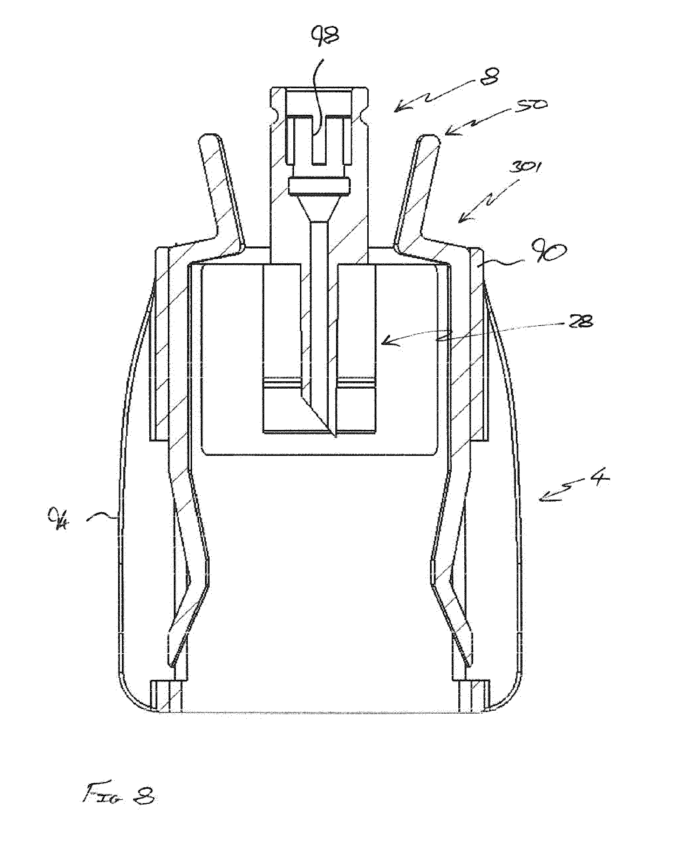

Figure 6a shows a kit of parts for a reconstitution and subsequent injection. The kit comprises a syringe 90 which contains a reconstitution liquid 94, a vial 92 containing a lyophilised medicament product 80, an adapter 201 as described above and a needle 220. The needle 220 includes a hub 24 and a shaft 22, the shaft 22 being a hollow rod, typically metal which an axial outlet and a sharpened tip. The shaft 22 is attached to the hub 24 which includes radial projections 96 which engage with corresponding recesses 98 (best shown in Figure 8) in the hub attachment portion of the adapter 210 to allow torque to be easily transmitted between the adapter 201 and the hub 24.

Figure 6b shows an assembly of the adapter 201 and the needle 220. The needle 220 has been coupled to the hub attachment portion of the adapter 201 as described above. In some kits the adapter 201 and needle 220 may be supplied already coupled together.

Figure 6c shows the assembly of Figure 6b with the vial 92 inserted into the container attachment portion of the adapter 201 ready to have a syringe 90 attached to the needle hub to begin reconstitution as shown in Figure 5.

Figure 7 shows a syringe 90 and needle 220 assembly ready for injection following the reconstitution steps of Figure 5. The syringe 90 and needle 220 assembly is removed from the adapter 201 which makes the needle 220 available for an injection. In some embodiments, for example an autoinjector, the needle may remain hidden within the body of the device until an injection operation is activated. Since the needle 220 has not been used to perform a piercing operation prior to its first use for injection the needle tip will not have been dulled and any silicone coating applied will not have been removed so the patient should experience no pain in excess of that which would be expected if a new needle was used for the injection.

Figure 8 shows a further embodiment of an adapter 301. In order to facilitate moulding the adapter comprises an inner part 92 and a sleeve part 90. The inner part 92 comprises the flags, snap fit features 28, the hub attachment portion 8 and the container attachment portion 4 and is substantially skeletal in form. The sleeve part 90 snap fits to the inner part and provides external handling ridges 94 similar to those seen on previous embodiments which facilitate a user rotating the adapter 301. The sleeve 90 also provides some additional rigidity to the adapter 301 and provides a non skeletal surface for a user to handle.

It should be understood that the invention has been described above by way of example only and that modifications in detail can be made without departing from the scope of the claims.

## Claims

1. An adapter, the adapter comprising a base having a container attachment portion (4) on a first side (6) thereof and a needle hub attachment portion (8) on a second side (10) thereof, the first side (6) being opposite the second side (10), the adapter including a puncturing member (12) extending along a first axis (14) for entering a container (40) inserted into the container attachment portion (4), the adapter including a channel (15) extending substantially along the first axis (14) from the needle hub attachment portion (8), through the base (2) and along the puncturing member (12) to an outlet (16) at, or adjacent, a tip of the puncturing member (12), the adapter configured such that a needle comprising a shaft (22) extending from a hub (24) can be inserted into the adapter such that the hub (24) engages the hub attachment portion (8) and the shaft (22) extends through the base (2) and within the channel (15), the outlet (16) is an axial outlet from the puncturing member (12), the axial outlet (16) extending substantially along the first axis (14) and configured such that
the needle shaft (22) extends within the channel (15) towards the axial outlet (16), the container attachment portion (4) is adapted to snap fit to a vial (44) and the puncturing member (12) is sharpened such that it will penetrate an elastomeric vial stopper (46) sealing the vial (44) as the vial (44) is inserted into the container attachment portion (4) and
**characterized in that**
the needle hub attachment portion (8) is adapted to releasably couple to a needle hub (24) inserted therein, the needle hub attachment portion (8) including a seal (26) for sealing to the needle hub (24) inserted therein.

2. An adapter as claimed in claim 1, in which the adapter includes a resiliently biased flag (50), the adapter is arranged such that the flag (50) is biased to a first position when no container (40) is coupled to the adapter and which is forced to a second position when a container (40) is coupled to the container attachment portion (4).

3. An adapter assembly comprising a needle and an adapter, the adapter being as claimed claim 1 or claim 2, and the needle (20) comprising a hub (24) and a shaft (22), the shaft extending from the hub (24), the needle (20) being suitable for performing a subcutaneous injection when coupled to a syringe, the needle hub (24) being releasably coupled to the need hub attachment portion (8) of the adapter.

4. A method of reconstituting a medicament comprising providing a container (40) containing a medicament to be reconstituted, a syringe containing a liquid, a needle (20) comprising a hub (24) and a shaft (22), the hub (24) being coupled to the syringe and an adapter as claimed in claim 1 or claim 2 the method comprising the steps of:
arranging the needle (20) within the adapter such that the hub (24) engages the hub attachment portion (8) and the shaft (22) extends through the base (2) and within the channel (15) towards the outlet (16);
coupling the container (40) to the container attachment portion (4) such that the puncturing member (12) enters the container (40) to create a reconstitution assembly;
using the syringe to force fluid through the needle shaft (22) to cause liquid to spray from the shaft (22), through the axial outlet (16) in the puncturing member (12) and into the container (40).

5. A method as claimed in claim 4, in which, after spraying the liquid into the container the method further includes, the steps of:
arranging the reconstitution assembly with the container (40) above the needle (20);
using the syringe to draw the solution from the container (40) into the syringe;
using the syringe to force the solution back into the container (40) through the needle (20).

6. A kit of parts for reconstituting and delivering a medicament, the kit comprising a container (40) containing a medicament to be reconstituted, a syringe containing a liquid, a needle (20) comprising a hub (24) and a shaft (22), the hub (24) being coupled to the syringe and an adapter, the adapter being as claimed In claim 1 or claim 2.

## Patentansprüche

1. Adapter, wobei der Adapter eine Basis mit einem Behälteranbringungsabschnitt (4) auf einer ersten Seite (6) davon und einen Nadelnabenanbringungssabschnitt (8) auf einer zweiten Seite (10) davon umfasst, wobei die erste Seite (6) der zweiten Seite (10) entgegengesetzt ist, wobei der Adapter ein Durchstechelement (12), das sich entlang einer ersten Achse (14) für die Eingabe eines Behälters (40), eingesetzt in den Behälteranbringungsabschnitt (4), erstreckt, einschließt, wobei der Adapter einen Kanal (15), der sich im Wesentlichen entlang der ersten Achse (14) von dem Nadelnabenanbringungsabschnitt (8), durch die Basis (2) und entlang dem Durchstechelement (12) zu einem Auslass (16) an oder neben der Spitze des Durchstechelements (12) erstreckt, einschließt, der Adapter so konfiguriert, dass eine Nadel mit einem Schaft (22), der sich von einer Nabe (24) aus erstreckt, in den Adapter eingeführt werden kann, so dass die Nabe (24) in Eingriff mit dem Nabenanbringungssabschnitt (8) ist und der Schaft (22) sich durch die Basis (2) und innerhalb des Kanals (15) erstreckt, der Auslass (16) ein axialer Auslass von dem Durchstechelement (12) aus ist, wobei sich der axiale Auslass (16) im Wesentlichen entlang der ersten Achse (14) erstreckt und so konfiguriert ist, dass
der Nadelschaft (22) sich innerhalb des Kanals (15) zum axialen Auslass (16) hin erstreckt, der Behälteranbringungsabschnitt (4) zum Einschnappen mit einer Phiole (44) angepasst ist und das Durchstechelement (12) zugespitzt ist, so dass es einen elastomeren Phiolenstopper (46) zum Abdichten der Phiole (44) durchdringen kann, da die Phiole (44) in den Behälteranbringungsabschnitt (4) eingesetzt wird und **dadurch gekennzeichnet,**
**dass** der Nadelnabenanbringungsabschnitt (8) angepasst ist, um lösbar mit einer Nadelnabe (24), die darin eingesetzt ist, zu koppeln, wobei der Nadelnabenanbringungsabschnitt (8) eine Dichtung (26) zum Abdichten der Nadelnabe (24), die darin eingesetzt ist, einschließt.

2. Adapter nach Anspruch 1, wobei der Adapter eine elastische vorgespannte Flagge (50) einschließt, der Adapter so angeordnet ist, dass die Flagge (50) in einer ersten Position vorgespannt ist, wenn kein Behälter (40) mit dem Adapter gekoppelt ist, und die an eine zweite Position gezwungen wird, wenn der Behälter (40) an den Behälteranbringungsabschnitt (4) gekoppelt ist.

3. Adapteranordnung, die eine Nadel und einen Adapter umfasst, der Adapter Anspruch 1 oder Anspruch 2 entsprechend, und die Nadel (20) eine Nabe (24) und einen Schaft (22) umfassend, wobei sich der Schaft von der Nabe (24) aus erstreckt, wobei die Nadel (20) zum Durchführen einer subkutanen Injektion geeignet ist, wenn sie mit einer Spritze gekoppelt ist, wobei die Nadelnabe (24) lösbar an den Nadelnabenanbringungsabschnitt (8) des Adapters gekoppelt ist.

4. Verfahren zur Wiederherstellung eines Medikaments, umfassend das Bereitstellen eines Behälters (40) mit einem Medikament, das wiederhergestellt werden soll, eine Spritze mit einer Flüssigkeit, eine Nadel (20) mit einer Nabe (24) und einem Schaft (22), wobei die Nabe (24) mit der Spritze gekoppelt ist, und einen Adapter nach Anspruch 1 oder Anspruch 2, wobei das Verfahren folgende Schritte umfasst:
Anordnen der Nadel (20) innerhalb des Adapters, so dass die Nabe (24) in Eingriff mit dem Nadelnabenanbringungsabschnitt (8) ist und der Schaft (22) sich durch die Basis (2) und innerhalb des Kanals (15) zum Auslass (16) hin erstreckt;
Koppeln des Behälters (40) an den Behälteranbringungsabschnitt (4), so dass das Durchstechelement (12) in den Behälter (40) eintritt, um eine Wiederherstellungsanordnung zu schaffen;
durch Benutzung der Spritze das Fluid durch den Nadelschaft (22) zu zwingen, um Flüssigkeit herbeizuführen, um vom Schaft (22) aus durch den axialen Auslass (16) im Durchstechelement (12) und in den Behälter (40) zu sprühen.

5. Verfahren nach Anspruch 4, bei dem nach dem Sprühen der Flüssigkeit in den Behälter das Verfahren ferner folgende Schritte umfasst:
Anordnen der Wiederherstellungssanordnung mit dem Behälter (40) über der Nadel (20);
Benutzen der Spritze, um die Lösung vom Behälter (40) in die Spritze zu drücken; Benutzen der Spritze, um die Lösung zurück in den Behälter (40) durch die Nadel (20) zu zwingen.

6. Kit aus Teilen für die Wiederherstellung und die Bereitstellung eines Medikaments, wobei das Kit einen Behälter (40), der ein Medikament, das wiederhergestellt werden soll, eine Spritze mit einer Flüssigkeit, eine Nadel (20) mit einer Nabe (24) und einem Schaft (22) enthält, wobei die Nabe (24) mit der Spritze und einem Adapter gekoppelt ist, wobei der Adapter Anspruch 1 oder Anspruch 2 entspricht.

## Revendications

1. Adaptateur, l'adaptateur comprenant une base ayant une partie de fixation de conteneur (4) sur un premier côté (6) de celui-ci et une partie de fixation d'embase d'aiguille (8) sur un deuxième côté (10) de celui-ci, le premier côté (6) étant opposé au deuxième côté (10), l'adaptateur comportant un membre de crevaison (12) s'étendant le long d'un premier axe (14) pour entrer dans un conteneur (40) inséré dans la partie de fixation de conteneur (4), l'adaptateur comportant un canal (15) s'étendant sensiblement le long du premier axe (14) depuis la partie de fixation d'embase d'aiguille (8), à travers la base (2) et le long du membre de crevaison (12) jusqu'à une sortie (16) à, ou adjacent, une extrémité du membre de crevaison (12), l'adaptateur étant configuré de sorte qu'une aiguille comprenant une tige (22) s'étendant depuis une embase (24) peut être insérée dans l'adaptateur de sorte que l'embase (24) engage la partie de fixation d'embase (8) et que la tige (22) s'étend à travers la base (2) et au sein du canal (15), la sortie (16) est une sortie axiale depuis le membre de crevaison (12), la sortie axiale (16) s'étendant sensiblement le long du premier axe (14) et étant configuré de sorte que
la tige de l'aiguille (22) s'étend au sein du canal (15) vers la sortie axiale (16), la partie de fixation de conteneur (4) est adaptée pour s'encliqueter à un flacon (44) et le membre de crevaison (12) est affilé de sorte qu'il pénétrera un bouchon de flacon élastomère (46) scellant le flacon (44) tandis que le flacon (44) est inséré dans la partie de fixation de conteneur (4) et **caractérisé en ce que**
la partie de fixation d'embase d'aiguille (8) est adaptée pour s'accoupler de manière libérable à une embase d'aiguille (24) insérée dans celle-ci, la partie de fixation d'embase d'aiguille (8) comportant un sceau (26) pour sceller l'embase d'aiguille (24) insérée dans celle-ci.

2. Adaptateur selon la revendication 1, dans lequel l'adaptateur comporte un indicateur fermement biaisé (50), l'adaptateur est disposé de sorte que l'indicateur (50) est biaisé à une première position lorsqu'aucun conteneur (40) n'est accouplé à l'adaptateur et qui est forcé à une deuxième position lorsqu'un conteneur (40) est accouplé à la partie de fixation de conteneur (4).

3. Montage d'adaptateur comprenant une aiguille et un adaptateur, l'adaptateur étant selon la revendication 1 ou la revendication 2, et l'aiguille (20) comprenant une embase (24) et une tige (22), la tige s'étendant depuis l'embase (24), l'aiguille (20) étant apte à réaliser une injection sous-cutanée lorsqu'elle est accouplée à une seringue, l'embase d'aiguille (24) étant accouplée de manière libérable à la partie de fixation d'embase d'aiguille (8) de l'adaptateur.

4. Procédé de reconstitution d'un médicament comprenant l'approvisionnement d'un conteneur (40) contenant un médicament à reconstituer, une seringue contenant un liquide, une aiguille (20) comprenant une embase (24) et une tige (22), l'embase (24) étant accouplée à la seringue et à un adaptateur selon la revendication 1 ou la revendication 2 le procédé comprenant les étapes de :
disposer l'aiguille (20) au sein de l'adaptateur de sorte que l'embase (24) engage la partie de fixation d'embase (8) et que la tige (22) s'étend à travers la base (2) et au sein du canal (15) vers la sortie (16) ;
accoupler le conteneur (40) à la partie de fixation de conteneur (4) de sorte que le membre de crevaison (12) entre dans le conteneur (40) pour créer un montage de reconstitution ;
utiliser la seringue pour forcer le fluide à travers la tige d'aiguille (22) pour jaillir le liquide de la tige (22), à travers la sortie axiale (16) au membre de crevaison (12) et dans le conteneur (40).

5. Procédé selon la revendication 4, dans lequel, après avoir jailli le liquide dans le conteneur le procédé comporte en outre, les étapes de :
disposer le montage de reconstitution avec le conteneur (40) au-dessus de l'aiguille (20) ;
utiliser la seringue pour faire venir la solution depuis le conteneur (40) dans la seringue ;
utiliser la seringue pour forcer la solution à rentrer dans le conteneur (40) à travers l'aiguille (20).

6. Kit de pièces pour reconstituer et délivrer un médicament, le kit comprenant un conteneur (40) contenant un médicament à reconstituer, une seringue contenant un liquide, une aiguille (20) comprenant une embase (24) et une tige (22), l'embase (24) étant accouplée à la seringue et à un adaptateur, l'adaptateur étant selon la revendication 1 ou la revendication 2.
